# EUROPEAN PATENT SPECIFICATION

(11) **EP 2 800 545 B1**
(45) Date of publication and mention of the grant of the patent: **27.07.2016**
(21) Application number: 12700037.0
(22) Date of filing: 04.01.2012
(51) Int. Cl.: A61M 5/14, F16M 11/00, F16M 13/02, A61G 12/00, A61G 13/10, A61M 5/145

(54) **RACK FOR HOLDING MEDICAL DEVICES**
HALTEGESTELL FÜR MEDIZINISCHE VORRICHTUNGEN
CRÉMAILLÈRE DESTINÉE À MAINTENIR DES DISPOSITIFS MÉDICAUX

(43) Date of publication of application: 12.11.2014
(73) Proprietor: Fresenius Vial SAS, 38590 Brézins (FR)
(72) Inventor: WOLFF, Rémy, F-38210 Morette (FR); PONCON, Gilbert, F-38340 Pommiers la Placette (FR); ARCHAT, Damien, F-38000 Grenoble (FR)
(74) Representative: Kusche, Robert
(86) International application number: PCT/EP2012/050107
(87) International publication number: WO 2013/102494

(56) References cited:
- EP-A1- 1 647 251
- EP-A1- 1 837 048
- EP-A1- 2 058 911
- DE-A1- 19 748 480
- US-A- 5 306 109
- US-A- 5 829 723
- US-A1- 2003 037 375
- US-A1- 2008 149 788

## Description

The invention relates to a rack for holding at least one medical device according to the preamble of claim 1, a medical device attachable to a rack according to the preamble of claim 12 and an arrangement of a rack and a medical device according to claim 13.

A rack of this kind comprises at least one first connection element to engage with a second connection element of a medical device for connecting the medical device to the rack. The at least one first connection element of the rack herein can be connected with the second connection element of the medical device by attaching the medical device in an engagement direction to the rack.

Racks of this kind serve to fixedly hold and organize medical devices such as infusion pumps to administer fluids, for example medication or nutrients, to a patient in a hospital environment. Such racks in general comprise multiple connection elements to hold multiple medical devices such that the medical devices via the rack can be organized in a space efficient manner for example by forming a vertical stack of medical devices. The rack herein serves as a communication spine providing a communication among the medical devices attached to the rack and between the medical devices and external periphery devices such as a nurse call, a printer, a computer, a bar code reader or an external communication network. Further, the rack serves to supply the medical devices attached to the rack with power.

Commonly, for connecting a medical device such as an infusion pump to the rack the medical device is mounted in an engagement direction to the rack, for example by inserting the medical device into a slot of the rack, to bring connection means of the rack into engagement with connection means of the medical device. In general, in racks known today several connection elements for establishing a mechanically reliable connection capable of holding the weight of the medical device and fixing the medical device in a secure manner to the rack on the one hand and for establishing an electrical connection to provide a communication link and a power link to the medical device on the other hand are used. Such connection elements, on the one hand for providing a mechanical connection and on the other hand for providing an electrical connection, are separate, wherein an electrical connection element may be used to at the same time provide a power link and a communication link.

Because different components are used to provide different (mechanical and electrical) connecting functions, it is necessary to arrange the connecting components on the rack and the medical device such that it is ensured that both the mechanical connection and the electrical connection are safely established when the mechanical device is attached to the rack. If for example the electrical connection is not fully established when the medical device is mechanically fixed to the rack, there is a risk that the medical device cannot be supplied with power or a communication may not work such that the functioning of the medical device cannot be guaranteed. Further, because multiple connections must be established, the installation of medical devices to the rack may be cumbersome and may involve several steps, bearing the risk to forget a fixing step when attaching a medical device to a rack, with possibly severe consequences if for example an electrical connection is not fully established.

In addition, medical devices of this kind may be conceived to be complex to handle, and it may be difficult to clean such medical devices because connection elements may be small and may not be easily accessible due to cavities and grooves or hooks of a connection element. A cleaning of the medical devices for example in the environment of an intensive care unit of a hospital is absolutely necessary to fulfil hygienic requirements.

US 2001/0044602 A1 discloses an infusion pump comprising a mobile self-contained functional basic module with a pump portion and an energy storage means. The mobile basic module can be inserted into an operating module which comprises an input device for a complex programming of the infusion pump.

EP 0 477 551 B1 discloses a rack to which multiple medical devices in the shape of infusion pumps may be connected. Different connection elements are provided to establish both a mechanical connection and an electrical connection, wherein mechanical connection elements serving to mechanically hold and fix a medical device are spatially separated from an electrical connection element in the shape of a multi-channel coupling element.

It is an object of the instant invention to provide a rack, a medical device and an arrangement including a rack and a medical device which allow for an easy, yet reliable and secure electrical and mechanical connection between a medical device and a rack.

This object is achieved with a rack comprising the features of claim 1.

Accordingly, within the rack the at least one first connection element of the rack is constituted to establish a mechanical connection for mechanically fixing the medical device to the rack and an electrical connection for electrically connecting the medical device to the rack.

The instant invention is based on the idea to use a single connection element to both establish a mechanical connection and an electrical connection between the rack and a medical device to be attached to the rack. The rack, for this purpose, comprises a first connection element which can be engaged with a second connection element of the medical device, wherein one of the first connection element and the second connection element may be a male connector and the other of the first connection element and the second connection element may be a female connector, the male connector being engagable into the female connector.

Via a single connection element on the rack, hence, a medical device can be both electrically and mechanically fixed to the rack. The first connection element of the rack serves to hold and fix the medical device to the rack such that the attachment of the medical device to the rack mechanically is provided via the first connection element. In addition, the first connection element also establishes the electrical connection between the medical device and the rack, wherein the electrical connection may include both a power connection for supplying the medical device with electric power and a communication link for establishing a communication connection between the medical device and the rack.

Preferably, the rack comprises a plurality of (first) connection elements, for example four (first) connection elements, such that four medical devices can be attached to the rack. Each first connection element of the rack herein may be shaped either as a connection cone protruding from a body of the rack to engage with a connection opening of the second connection element of the medical device or as a connection opening to engage with a connection cone of the second connection element of the medical device. In the first case the first connection element of the rack represents a male connector in the shape of a connection cone to engage with a female connector on the side of the medical device. In the second case the first connection element of the rack is a female connector into which a male connector on the side of the medical device can be inserted. In general, there is no functional difference whether the male connector is arranged on the rack and the female connector on the medical device or vice versa.

The first connection element of the rack and the second connection element of the medical device have a rotationally symmetric shape about a rotational symmetry axis directed along the engagement direction. If the first connection element of the rack is shaped as a protruding connection cone, this connection cone is rotationally symmetric about the engagement direction such that a medical device with its second connection element in the shape of a connection opening can be plugged onto the connection cone of the first connection element of the rack by attaching the medical device in the engagement direction to the rack.

By giving the first connection element and the second connection element, a rotationally symmetric shape - or at least by giving the first connection element and the second connection element a rotationally periodic shape, which does not form part of the present invention (for example a rectangular or quadratic shape in cross section perpendicular to the engagement direction) -, it may be possible to attach the medical device in different engagement positions to the rack, the different engagement positions corresponding to different angular positions when turning the medical device about the engagement direction. For example, the first connection element of the rack and the second connection element of the medical device may be constituted mechanically and electrically such that the medical device may be attached in two different engagement positions to the rack, wherein in a second engagement position the medical device is turned by a 180° about the engagement direction compared to a first engagement position. It however is also conceivable to constitute the first connection element of the rack and the second connection element of the medical device such that the medical device may be attached in more than two, for example in four different engagement positions to the rack.

The first connection element of the rack and correspondingly the second connection element of the medical device may comprise a plurality of electrical contacts, wherein different contacts may serve for different purposes (for example to establish a power connection or a communication link) or may be used in a multiplexed fashion for multiple purposes (for example for both establishing a power connection and a communication link). For example, the first connection element and the second connection element may comprise four electrical contacts which are spaced evenly (90°) apart about the engagement direction on a surface of the first connection element and, respectively, the second connection element. By spacing the electrical contacts evenly apart it may be provided that the medical device can be attached in different engagement positions to the rack. Instead of spacing all electrical contacts evenly apart about the engagement direction, it also is possible to group the electrical contacts to distinguished groups, wherein the groups of the electrical contacts are spaced evenly apart.

The first connection element of the rack and the second connection element of the medical device establish both a mechanical connection and an electrical connection. To ensure that a medical device is securely held on the rack, the first connection element and the second connection element may comprise mechanical locking means for mechanically locking the at least one first connection element of the rack to the second connection element of the medical device. For this, the first connection element of the rack, as a mechanical locking means, may for example comprise a groove to engage with at least one engagement element of the second connection element of the medical device. Vice versa, it is also possible that the first connection element of the rack comprises at least one engagement element to engage with a groove of the second connection element of the medical device.

The one engagement element may for example be a ball arranged on the associated connection element, which may be brought into engagement with the groove on the other connection element, such that the mechanical connection between the first connection element and the second connection element is locked once the ball engages the groove.

The at least one engagement element in the shape of a ball for example may be arranged on the associated connection element in a pretensioned manner, such that the connection elements of the rack and the medical device may be attached to each other and the at least one engagement element snaps into engagement with the groove once the connection elements are plugged fully into each other. The at least one engagement element may then be locked in its engaging position such that the connection of the connection elements cannot be released without releasing the locking.

The rack, in a further embodiment, may comprise a protruding section extending vertically to the engagement direction on the rack to engage with an associated indentation of the medical device. Multiple connection elements of the rack may herein be arranged on the protruding section. The protruding section may serve to define two engagement positions (offset by 180° about the engagement direction) to engage a medical device to the rack, such that a user intuitively knows in what positions the medical device may be attached to the rack.

The first connection element of the rack and the second connection element of the medical device serve to establish both a mechanical connection and an electrical connection, the electrical connection preferably including both a power connection and a communication link. Further, the first connection element of the rack may comprise a detection device for detecting whether a medical device is present and connected to the first connection element or not. The detection device herein may be constituted to detect if a medical device is present at the specific first connection element at all. The detection device further may be constituted to detect whether the mechanical and electrical connection between the first connection element of the rack and the second connection element of the medical device is established in a correct and functional manner.

The object underlying the invention is also achieved by a medical device attachable to a rack for holding at least one medical device, the medical device comprising a second connection element to engage with a first connection element of the rack for connecting the medical device to the rack, wherein the second connection element of the medical device is connectable with the first connection element of the rack by attaching the medical device in an engagement direction to the rack. The medical device is characterized in that the second connection element of the medical device is constituted to establish a mechanical connection for mechanically fixing the medical device to the rack and an electrical connection for electrically connecting the medical device to the rack.

The advantageous embodiments described above for the rack similarly are applicable also for the medical device.

The object is further achieved by an arrangement of a rack of the kind described above and a medical device of the kind as described above.

In a specific embodiment of such an arrangement, the first connection element (of the rack) and the second connection element (of the medical device) each comprise at least two electrical contacts, wherein in the attached state of the rack and the medical device via the at least two electrical contacts both a low speed data connection and a high speed data connection between the medical device and the rack is established. Hence, the (first) connection element of the rack and the (second) connection element of the medical device each have two or more electrical contacts, which are contacting each other when the medical device is attached to the rack and, hence, the connection element of the rack engages the connection element of the medical device. This is based on the idea to use the at least two electrical contacts to provide both a low speed data connection and a high speed data connection via the same lines. Via the at least two electrical contacts at least two electrical lines are established, and via such lines both a low speed data communication and a high speed data communication - using the same lines - may take place when the medical device is attached to the rack.

Thus, by using the same lines for a high speed communication and a low speed communication the overall number of contacts to be provided within the (first) connection element of the rack and the (second) connection element of the medical device is reduced, thus reducing the complexity of the overall connection and also reducing the risk of breakage of the connection.

Via the at least two electrical contacts of the (first) connection element of the rack and the (second) connection element of the medical device in addition also a power supply connection, in particular a direct current connection, may be established. Via the same lines, thus, a high speed communication, a low speed communication and a power supply may take place, hence using the same lines to provide different communication means and the power supply for the medical device.

The low speed data connection may have for example a data rate equal to or smaller than 1 kbit/s and may use a signal frequency in the kHz range. In contrast, the high speed data connection may have a data rate equal to or larger than 2 Mbit/s, preferably 10 Mbit/s or even 100 Mbit/s and may use a signal frequency in the MHz or GHz range. The high speed data connection may for example be an Ethernet connection, for example according to the IEEE802.3 standard.

The idea of the invention shall subsequently be described in more detail according to the embodiments shown in the figures. Herein,
- Fig. 1: shows a schematic overview of a rack for holding medical devices, in particular infusion pumps, being placed at a bedside of a patient;
- Fig. 2: shows a perspective overview of a rack comprising a base part and a holding part detachably connected to the base part;
- Fig. 3A: shows a schematic view of the holding part and the base part connected to each other in a normal state of use of the rack;
- Fig. 3B: shows a schematic view of the holding part and the base part in a detached state;
- Fig. 4: shows a schematic view illustrating circuitry of the base part and the holding part;
- Fig. 5: shows a schematic view of the holding part and the base part in a normal state of use of the rack, the holding part including a fastening means for attaching a fluid container to the holding part;
- Fig. 6: shows a perspective view of the holding part comprising a number of first connection elements to be engaged with a second connection element of a medical device;
- Fig. 7: shows a perspective view of a first connection element in the shape of a male connector and a second connection element in the shape of a female connector in a detached fashion;
- Fig. 8: shows a partially cut view of a first connection element on the side of the rack attached to a second connection element on the side of the medical device;
- Fig. 9A: shows a schematic view of an electrical connection of a medical device to a rack in a first engagement position;
- Fig. 9B: shows a schematic view of an electrical connection of a medical device to a rack in a second engagement position;
- Fig. 10: shows a circuit diagram of an electrical connection between a medical device and a rack;
- Fig. 11: shows a circuit diagram of a low speed data connection of the electrical connection between the medical device and the rack; and
- Fig. 12: shows a circuit diagram of a power and low speed data feeding over high speed data lines.

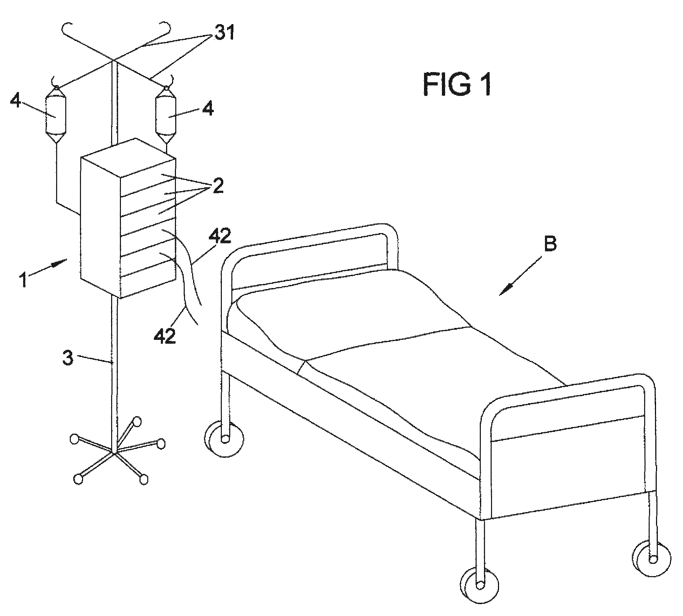

Fig. 1 shows in a schematic drawing a scenario as it typically can be found in a hospital environment, for example an intensive care unit of a hospital. Next to the bed B of a patient a number of medical devices 2 constituted as infusion pumps, such as syringe pumps or volumetric pumps, are located and connected to a patient via infusion lines 42. Such medical devices 2 serve to administer a fluid such as medication or nutrients for example contained in containers 4 via infusion lines 42 to the patient, such infusion lines 42 (especially in the environment of an intensive care unit of a hospital) possibly being vital to the patient such that they under all conditions must remain connected to the patient to ensure the required administration of medication, nutrients or the like.

Typically, such medical devices 2 constituted as infusion pumps are organized in a rack 1 to form a vertical stack of medical devices 2 which is fixed for example to a stand 3. The stand 3 may comprise wheels such that the stand 3 at least to some extend is movable with respect to the patient's bed B or together with the patient's bed B. The stand 3 may have the shape of a post to which the rack 1 for carrying the medical devices 2 is attached and comprises, at its top end, fastening means 31 in the shape of hooks to fasten a number of containers 4 containing medication or nutrients or other fluids to be administered to the patient.

The rack 1 serves to arrange the medical devices 2 in an organized fashion at the bedside of the patient. The rack 1 herein provides a power supply for the medical devices 2, ensures a secure and reliable fixation of the medical devices 2, and provides a communication of the medical devices 2 among each other and with an external communication network and with external periphery devices such as a nurse call, a printer, a computer, a monitor or the like.

Conventionally, the medical devices 2 can be fixed to the rack 1 and for this are mechanically and electrically connected to the rack 1 such that via the rack 1 each medical device 2 can be supplied with power and may communicate with other medical devices 2 and with external devices and/or an external communication network. The rack 1 hence serves as a communication spine providing a communication facility and an electric power supply and embedding the medical devices 2 into a hospital environment including a hospital communication network and a hospital management system.

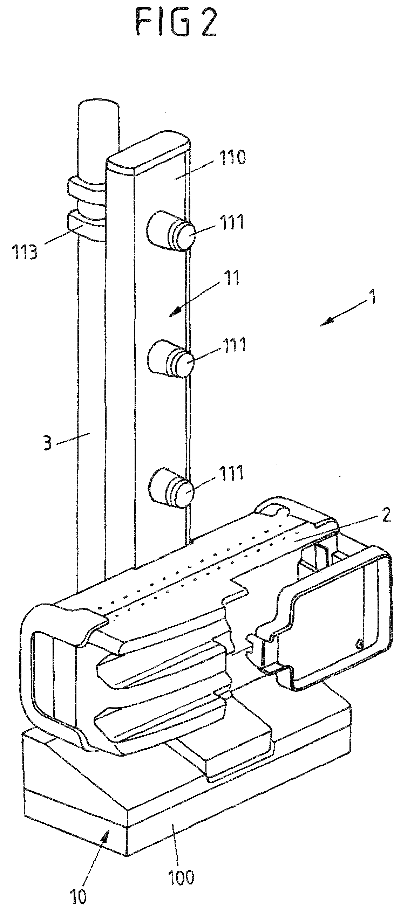

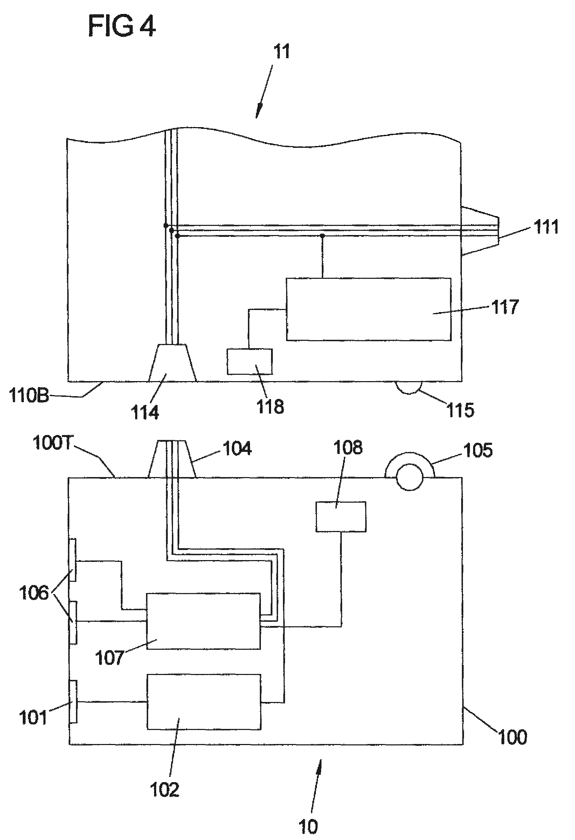

Fig. 2 to 4 show an embodiment of a rack 1 which comprises a base part 10 and a holding part 11 which is detachable from the base part 10. Herein, the holding part 11 comprises a number of connection means in the shape of cone-shaped connection elements 111 arranged on a body (vertical column) 110 of the holding part 11. The holding part 11 may for example comprise four connection elements 111 for connecting four medical devices 2 in the shape of infusion pumps to the body (column) 110, yet other numbers of connection elements 111 such as six or eight being equally conceivable. The base part 10, to which the holding part 11 is connected, comprises in a housing 100 a power supply unit 102 (see Fig. 4) and a communication means 107 and thus serves to provide a communication and power interface to external devices, an external communication network and an external power supply.

Hence, the rack 1 is functionally divided into two parts, namely the base part 10 and the holding part 11. The holding part 11 with its connection elements 111 herein serves for mechanically fixing and holding the medical devices 2 and does not comprise a large and heavy power supply unit (including a transformer) or extensive communication circuitry and interfaces for connecting external devices. In a particular embodiment, the holding part 11 may even comprise no power supply and communication electronics at all, but comprises only connecting lines for connecting external devices 2 to the circuitry of the base part 10. The base part 10, in contrast, comprises all major electronic components that are necessary for providing communication and electric power to the medical devices 2 attached to the rack 1. In particular, the base part 10 comprises the power supply unit 102, in general including a transformer, and the communication means 107 (see Fig. 4). Further, the base part 10 comprises a number of communication ports 106 for connecting different periphery devices such as a nurse call, a barcode reader, a computer, a monitor, a printer or an external network or the like to the base part 10 and hence the rack 1. For this, different lines may be connected to the communication ports 106 which may for example be constituted as USB connections or Ethernet / LAN connections. The base part 10 further comprises a power connection 101 to which an external power line can be connected.

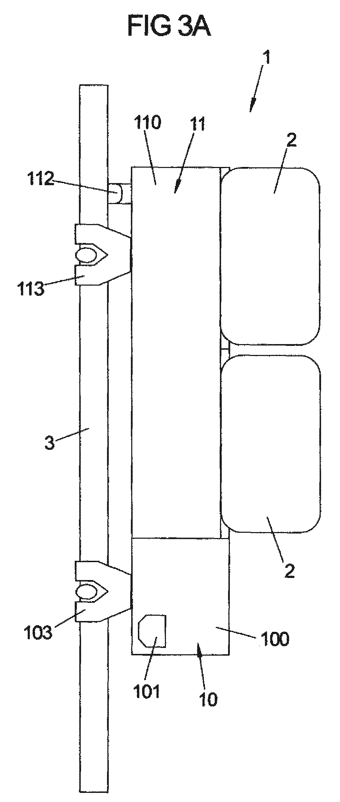

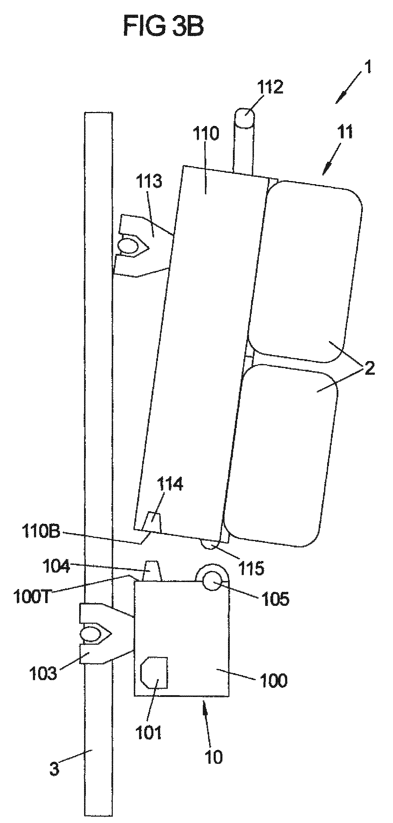

As indicated in Fig. 3A and 3B, the holding part 11 can be detached from the base part 10 to carry the holding part 11 together with medical devices 2 attached to the holding part 11 independent from the base part 10. Both the holding part 11 and the base part 10 herein comprise a fixing means, possibly constituted as a screw clamp, a spring-tensioned clamp or a hook, for fixing the holding part 11 and the base part 10 to the stand 3. To connect the holding part 11 to the base part 10 connection means 114, 115 on a bottom side 110B of the holding part 11 and connection means 104, 105 on a top side 100T of the housing 100 of the base part 10 are provided which serve to mechanically and electrically connect the holding part 11 to the base part 10. One connection means 104, 114 herein may be constituted to provide both a mechanical and electrical connection, wherein the electrical connection includes a communication link as well as a power supply connection. The other connection means 105, 115 may serve for providing a mechanical connection only. (It is also conceivable that both connection means 104, 114, 105, 115 provide both an electrical connection and a mechanical connection.)

When detaching the holding part 11 from the base part 10, the fixing means 113 of the holding part 11 is released from the stand 3 and the connections via the connection means 104, 114, 105, 115 are disconnected. The holding part 11 comprises a handle 112 which, in the pivoting position shown in Fig. 3B, can be used to carry the holding part 11 together with the medical devices 2 attached thereto. The handle 112 is pivotably connected to the body (column) 110 of the holding part 11 and may also cause a locking or unlocking of the connection of the holding part 11 via the fixing means 113 to the stand 3 and/or via the connection means 104, 114, 105, 115 to the base part 10.

Namely, in a rest position as shown in Fig. 3A the handle 112 may cause the fixing means 113 and the connection means 104, 114, 105, 115 to be locked such that the holding part 11 cannot be detached from the base part 10 and the stand 3. When pivoting the handle 112 into a move position as indicated in Fig. 3B, the fixing means 113 and the connection means 104, 114, 105, 115 may be unlocked, such that the holding part 11 can be detached from the base part 10 and the stand 3 and can be moved without the base part 10 and the stand 3.

When moving the holding part 11 without the base part 10 and the stand 3, the medical devices 2 attached to the holding part 11 may be power supplied by internal batteries of the medical devices 2. In addition, electric power may be provided to the medical devices 2 via a battery 117 (see Fig. 4) of the holding part 11 such that a continuous power supply of the medical devices 2 is ensured over a sufficient period of time when the holding part 11 is detached from the base part 10.

When the holding part 11 is detached from the base part 10, the holding part 11 may communicate via the base part 10 via a wireless communication interface 118 (see Fig. 4) establishing a connection to a wireless communication interface 108 of the base part 10 or an external communication network such as a wireless local area network (WLAN). Via the wireless communication interface 118, thus, a basic communication for the holding part 11 with its attached medical devices 2 may be provided to communicate control commands to the medical devices 2 or to exchange alerts or measurement values between an external network and/or the base part 10 and the medical devices 2 attached to the holding part 11.

By moving the holding part 11 together with the attached medical devices 2 without the base part 10 and the stand 3 the weight of the arrangement to be moved is substantially reduced compared to moving the full rack 1, and because the dimensions of the holding part 11 are small compared to the full rack 1 and the stand 3, the handling of the holding part 11 with the attached medical devices 2 is simplified compared to the handling of the full rack 1. Further, no lines must be disconnected when moving the holding part 11 since all lines can remain connected to the base part 10. In addition, the medical devices 2 remain in an organized fashion on the holding part 11 and via the holding part 11 may for example be attached to a bed B (for example by using the fixing means 113) to be moved in an easy way together with the bed B.

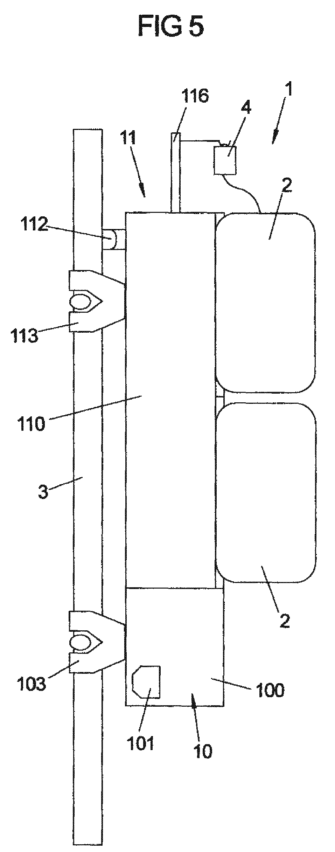

When moving the medical devices 2, in particular when they are constituted as volumetric infusion pumps, containers 4 containing a fluid such as medication or nutrients or the like to be administered to a patient must be moved together with the medical devices 2. Hence, in an embodiment shown in Fig. 5 the holding part 11 comprises a fastening means 116, possibly including one or multiple hooks, for fastening one or multiple containers 4 such as bottles or bags to the holding part 11 such that the containers 4 may easily be moved together with the holding part. The fastening means 116 may have the shape of a post protruding vertically from the body 110 of the holding part 11 and carrying one or multiple hooks.

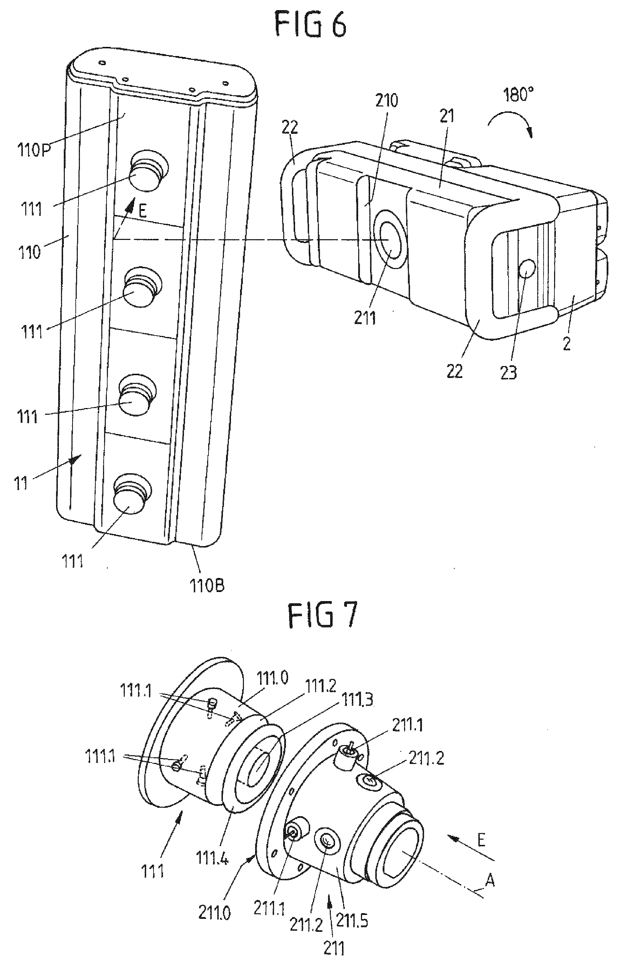

With reference to Fig. 6 to 8 subsequently the connection elements 111 of the rack 1 providing connection means to connect a medical device 2 to the holding part 11 of the rack 1 shall be explained in detail.

As visible in Fig. 6, on a protruding section 110P of the holding part 11 four (first) connection elements 111 - spaced apart along the protruding section 110P - are arranged each of which is constituted to engage with a (second) connection element 211 arranged within an indentation 210 on a backside of a housing 21 of a medical device 2. The medical device 2, constituting an infusion pump such as a syringe pump or a volumetric pump, can be attached to the rack 1 by plugging the second connection element 211 in an engagement direction E onto the first connection element 111 on the holding part 11 of the rack. For this, the medical device 2 can be grabbed on a handle 22 and can be attached to the rack 1 in the engagement direction E until the first connection element 111 of the rack 1 fittingly engages the second connection element 211 of the medical device 2.

The first connection element 111 and the second connection element 211 provide connection means which serve for establishing both a mechanical connection and an electrical connection between the rack 1 and the medical device 2. In the exemplary embodiment of Fig. 6 to 8 the first connection element 111 of the holding part 11 of the rack 1 is shaped as a male connector to be engaged with the second connection element 211 on the side of the medical device 2 in the shape of a female connector.

Although the connection elements 111, 211 will subsequently be described with reference to the connecting of a medical device 2 to the holding part 11 of the rack 1, it is to be noted that identical or at least similar connection elements can also be used to form the connection means 104, 114, 105, 115 for attaching the holding part 1 to the base part 10. In particular, the base part 10 may comprise a first connection element and the holding part 11 may comprise a second connection element, the first and the second connection element being constituted to establish a mechanical connection for mechanically fixing the holding part 11 to the base part 10 and an electrical connection for electrically connecting the holding part 11 to the base part 10.

In addition it shall be emphasized that the use of such connection elements 111, 211 is in principle not limited to a rack 1 as it has been described with reference to Fig. 2 to 4, but rather the connection elements 111, 211 may also be used in connection with any other type of rack.

As visible from Fig.7, the first connection element 111 comprises a connection cone 111.0 which fittingly may be inserted into a connection opening 211.0 of the second connection element 211. On the connection cone 111.0 of the first connection element 111 four electrical contacts 111.1 are arranged, which in a connected state of the first connection element 111 and the second connection element 211 are electrically contacting four electrical contacts 211.1 on a wall 211.5 of the second connection element 211. The electrical contacts 111.1 of the first connection element 111 as well as the electrical contacts 211.1 of the second connection element 211 are spaced evenly (90°) apart about a rotational axis of symmetry A pointing along the engagement direction E and forming a rotational symmetry axis of the connection cone 111.0 as well as of the engagement opening 211.0.

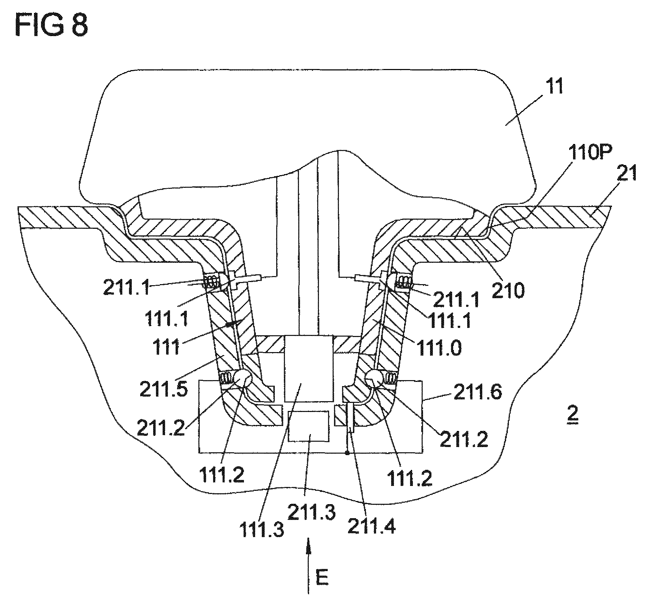

As can be seen from the partially cut view of Fig. 8, the electrical contacts 211.1 are arranged on the wall 211.5 of the second connection element 211 in a pretensioned manner, wherein the electrical contacts 211.1 can be moved by some distance perpendicularly to the engagement direction E. For pretensioning the electrical contacts 211.1, for example a spring is provided for each electrical contact 211.1 asserting a pretensioning force perpendicularly to the engagement direction E in a direction pointing radially inwards onto the associated electrical contact 211.1.

When connecting the second connection element 211 to the first connection element 111 by inserting the connection cone 111.0 into the connection opening 211.0, the electrical contacts 211.1 of the second connection element 211 slidingly get in touch with the electrical contacts 111.1 of the first connection element 111 and are slightly pushed outwards in the radial direction, such that the electrical contacts 211.1 of the second connection element 211 abut the electrical contacts 111.1 of the first connection element under an elastic pretension.

Because the electrical connection is established by slidingly bringing the electrical contacts 211.1 of the second connection element 211 into contact with the electrical contacts 111.1 of the first connection element 111, at the time of establishing the electrical connection also a cleaning of the contacts 111.1, 211.1 due to the sliding movement of the electrical contacts 111.1, 211.1 with respect to each other is achieved.

At the tip of the connection cone 111.0 of the first connection element 111a groove 111.2 having a toric shape is arranged which serves to establish a mechanically locked connection to fix the first connection element 111 in a secure manner to the second connection element 211. For this, four engagement elements 211.2 in the shape of balls are arranged on the wall 211.5 of the second connection element 211, the engagement elements 211.2 engaging the groove 111.2 in the connected state of the first connection element 111 and the second connection element 211 as visible in the partially cut view of Fig. 8.

The engagement elements 211.2 in the shape of balls are, similar to the electrical contacts 211.1, arranged in an elastically pretensioned manner on the wall 211.5. For this, an elastic spring may be associated with each engagement element 211.2 pretensioning the engagement element 211.2 perpendicularly to the engagement direction E in a direction pointing radially inwards.

When the connection cone 111.0 of the first connection element 111 is fittingly brought into engagement with the connection opening 211.0 of the second connection element 211, the engagement elements 211.2 in the shape of the balls are first pressed elastically outwards, until they - under the action of the pretensioning springs - snap into engagement with the grove 111.2 of the first connection element 111.

As visible in Fig. 8, in the connected state a front side 111.4 of the connection cone 111.0 abuts a pin 211.4 arranged on a backside of the connection opening 211.0. Through a pushing action onto the pin 211.4 when inserting the connection cone 111.0 into the connection opening 211.0, a locking device 211.6 (shown only in principle in Fig. 8) is actuated causing the engagement elements 211.2 to be locked in their engagement position engaging the groove 111.2 of the first connection elements 111. Because of the locking of the engagement elements 211.2 in their engagement position the second connection element 211 cannot be detached (without an unlocking actuation of the locking device 211.6) from the first connection element 111 such that the second connection element 211 (and with it the medical device 2) in a secure and reliable manner is held at the first connection element 111 (and hence at the rack 1).

As visible in Fig. 6, on the housing 21 of the medical device 2 an unlocking button 23 may be arranged which can be actuated for releasing the locking device 211.6 and, hence, for taking the medical device 2 of the rack 1.

The medical device 2, in the embodiment shown in Fig. 6 to 8, may be attached to the rack 1 in two different engagement positions, the one engagement position corresponding to the other engagement position when turning the medical device by 180° about the engagement direction E. The medical device 2, as indicated by the arrow in Fig. 6, can be turned about the engagement direction E by 180° and can be connected to the rack 1 via the same connection elements 111, 211 achieving functionally the same mechanical and electrical connection.

As visible from Fig. 7, when turning the second connection element 211 by 180° about the engagement direction E with respect to the first connection element 111, all four electrical contacts 111.2, 211.2 of one connection element 111, 211 will abut electrical contacts 111.2, 211.2 on the other connection element 111, 211, wherein an appropriate electrical switching circuitry may provide for a desired functionality of the electrical connection (for example to provide a power connection and a communication link) independent on the engagement position of the medical device 2.

As visible from Fig. 7 and Fig. 8, the first connection element 111 and the second connection element 211 each comprise an additional communication port 111.3, 211.3, possibly constituted as an infrared (IRDA) communication port for providing an additional communication link between the connection elements 111, 211.

Into the communication port 111.3 of the first connection element 111 in addition a detection device may be integrated serving to detect whether a medical device 2 is attached to the respective connection element 111. The detection device may for example be a Hall sensor or a micromechanic switch detecting the presence of a second connection element 211 on the first connection element 111.

A detection device of this kind may be used to detect whether a medical device 2 is connected to a particular connection element 111 of the rack 1 or not. The detection device 111.3 further may be used to detect whether a mechanical and electrical connection between the second connection element 211 and the first connection element 111 is established in a functionally correct manner. According to a detection signal of the detection device, then, a communication between the medical device 2 attached to the respective connection element 111 of the rack 1 and the rack 1 may be initiated (if a medical device 2 is connected to the respective first connection element 111) or terminated (if a medical device 2 is disconnected from the respective first connection element 111).

With connection means as they are described here on the one hand an easy and secure mechanical fixing and positioning of a medical device 2 on a rack 1 may be achieved and on the other hand electric power may be supplied to a medical device 2 and a communication link may be established. The connection elements 111, 211 herein may provide for a detection of a medical device 2 on a rack 1, and may provide a beneficial cleanability. Because for connecting a medical device 2 to the rack 1 the medical device 2 with its connection element 211 merely needs to be plugged to a connection element 111 of the rack 1 thus establishing in a single step both the electrical connection and the mechanical connection via a single connection element 111 of the rack 1, the handling of the medical device 2 for connecting it to the rack 1 is easy and safe.

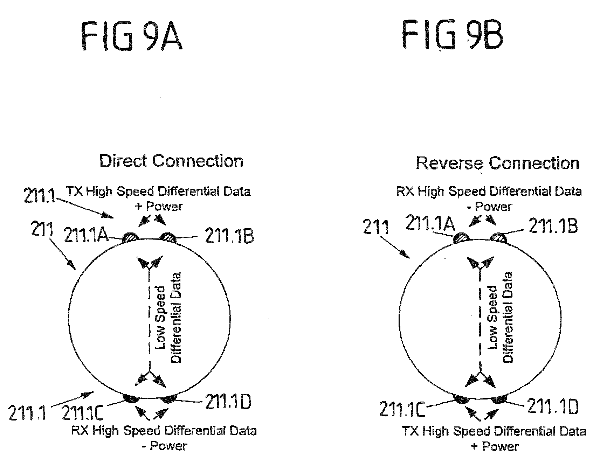

Figs. 9A, 9B to 12 show in schematic drawings an electrical connection as it is established between a medical device 2 and the rack 1 via the connection elements 111, 211 as shown in Fig. 7.

As described above, the connection elements 111, 211 each comprise four electrical contacts 111.1, 211.1 which, in a state in which the medical device 2 is attached to the rack 1, are in contact with each other and hence provide electrically conducting connection lines between the medical device 2 and the rack 1.

According to the embodiment of Figs. 9A, 9B to 12 the electrical connection established via the connection elements 111, 211 provides a high speed data connection, a low speed data connection and a power supply connection via the same four lines established via the four electrical contacts 111.1, 211.1 on the connection elements 111, 211. Hence, the electrical lines provided via the contacts 111.1, 211.1 are used to transmit high speed data at a large data rate, for example 10 Mbit/s, as well as low speed data at a fairly low data rate of for example 1 kbit/s and to feed an electrical power to supply the medical device 2.

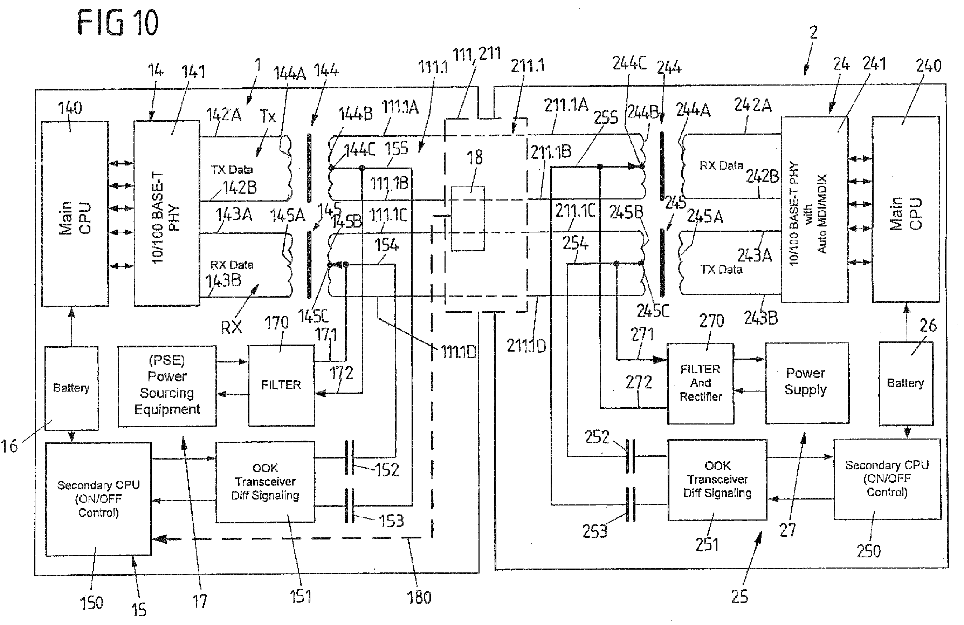

As shown in the circuit diagram of Fig. 10, both on the side of the rack 1 and on the side of the medical device 2 different electronic components are used to provide a high speed communication, a low speed communication and a power feed. For this, the rack 1 (for example contained in the base part 10 of the rack 1 as shown in Figs. 2 and 4) comprises a first communication means 14 with a first processor (main CPU) 140 and a communication interface 141 (for example an Ethernet communication interface). Further, a second communication means 15 providing a low speed communication, a battery 16 and a power sourcing equipment 17 to feed power to the medical device 2 are provided. Similarly, on the side of the medical device 2 a first communication means 24 with a first processor (main CPU) 240 and a communication interface 241 (for example an Ethernet communication interface), a second communication means 25, a battery 26 and a power supply 27 are provided.

Within the arrangement of the rack 1 and the medical device 2, the first communication means 14, 24 serve to provide a high speed data communication between the rack 1 and the medical device 2 via the connection elements 111, 211 used to electrically connect the medical device 2 to the rack 1. The second communication means 15, 25, in turn, serve to provide a low speed data communication, and the power sourcing equipment 17 on the side of the rack serves to feed power to the power supply 27 on the side of the medical device 2. As mentioned, the high speed data communication between the first communication means 14, 24 of the rack 1 and the medical device 2 as well as the low speed communication between the second communication means 15 of the rack 1 and the medical device 2 and the power feeding takes place over the same four lines established via the electrical contacts 111.1, 211.1 of the connection elements 111, 211.

For the high speed data communication, the first processor (main CPU) 140 of the rack 1 is connected via the communication interface 141 to the electrical contacts 111.1. For this, the communication interface 141 via lines 142A, 142B is connected to a primary winding of a first transformer 144 and via lines 143A, 143B to a primary winding 145A of a second transformer 145 of the rack 1. A secondary winding 144B of the first transformer 144 is connected to a first pair of electrical contacts 111.1A, 111.1 B of the four electrical contacts 111.1 of the connection element 111 of the rack 1, whereas a secondary winding 145B of the second transformer 145 is connected to a second pair of electrical contacts 111.1C, 111.1D of the four electrical contacts 111.1 of the connection element 111 on the side of the rack 1.

In a symmetric fashion, on the side of the medical device 2 the first processor (main CPU) 240 via the communication interface 241 is connected to the electrical contacts 211.1 of the connection element 211 on the side of the medical device 2. The communication interface 241 via a first pair of lines 242A, 242B is connected to a primary winding 244A of a first transformer 244, whose secondary winding 244B is connected to a first pair of contacts 211.1A, 211.1 B of the four electrical contacts 211.1 of the connection element 211 on the side of the medical device 2. In addition, the communication interface 241 via a second pair of lines 243A, 243B is connected to a primary winding 245A of a second transformer 245, whose secondary winding 145B is connected to a second pair of contacts 211.1C, 211.1D of the electrical contacts 211.1 of the connection element 211 on the side of the medical device 2.

Via the first pair of contacts 111.1A, 111.1 B, 211.1A, 211.1 B, in this way, a high speed differential transmit link TX and via the second pair of electrical contacts 111.1C, 111.1 D, 211.1C, 211.D a high speed differential receive link RX between the first processor 140 of the rack 1 and the first processor 240 of the medical device 2 is established, which are used to transmit high speed differential data signals from the rack 1 to the medical device 2 (transmit link TX) and from the medical device 2 to the rack 1 (receive link RX). Such differential data signals are transmitted in a differential way over the paired lines via the first pair of contacts 111.1A, 111.1 B, 211.1A, 211.1 B and via the second pair of contacts 111.1C, 111.1D, 211.1C, 211.1D, for example forming an Ethernet connection having a data rate of e.g. 10 Mbit/s or even higher. For this, the communication interfaces 141, 241 of the rack 1 and the medical device 2 may be for example formed as 10/100 base-T communication interfaces with a switchable data rate between 10 Mbit/s and 100 Mbit/s according to the Ethernet standard.

Within the electrical connection provided via the connection elements 111, 211 on the side of the rack 1 and on the side of the medical device 2, in addition the low speed data communication is provided using the second communication means 15, 25 on the side of the rack 1 and on the side of the medical device 2. Such second communication means 15, 25 comprise both on the side of the rack 1 and on the side of the medical device 2 a second processor 150, 250 and a transceiver 151, 251. The second processor 150, 250 via the respective transceiver 151, 251 is connected via a first capacitor 152, 252 and a first line 154, 254 to a center tap 145C, 245C of the secondary winding 145B, 245B of the second transformer 145, 245 of the rack 1 respectively the medical device 2 within the high speed differential receive link RX. The transceiver 151, 251 of the rack 1 respectively the medical device 2 via a second capacitor 153 and a second line 155 in addition is connected to a center tap 144C, 244C of the secondary winding 144B, 244B of the second transformer 144, 244 on the side of the rack 1 respectively the side of the medical device 2 within the high speed differential transmit link TX. Via the two lines 154, 155, 254, 255 a low speed differential link between the second processor 150 of the rack 1 and the second processor 250 of the medical device 2 over the first pair of contacts 111.1A, 111.1B, 211.1A, 211.1B and the second pair of contacts 111.1C, 111.1D, 211.1 C, 211.1 D is established. Differential signals are transmitted over the lines 154, 155, 254, 255, wherein the lines of the first pair of electrical contacts 111.1A, 111.1B, 211.1A, 211.1B and the lines of the second pair of electrical contacts 111.1C, 111.1D, 211.1C, 211.1 D are each used in their common mode.

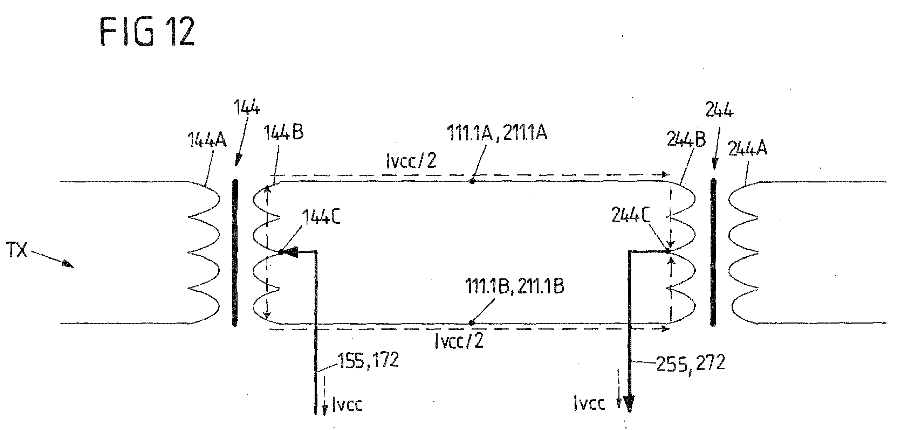

This is illustrated schematically in Fig. 12 according to the lines of the first pair of contacts 111.1A, 111.1B, 211.1A, 211.1B. Herein, the lines 155, 255 connected to the associated second processor 150, 250 are connected to the center tap 144C, 244C of the associated secondary winding 144B, 244B of the transformer 144, 244 of the rack 1 respectively the medical device 2. The center tap 144C, 244C is at a position of a virtual ground (with regard to differential signals transmitted over the transformer 144, 244) such that via the center tap 144C, 244C only the common mode of the lines connecting the secondary winding 144B of the transformer 144 of the rack 1 and the secondary winding 244B of the transformer 244 of the medical device 2 is excited.

Because the transformers 144, 244 (primarily) transmit only differential data signals the low speed data signals (exciting the common mode) are not transmitted via the transformers 144, 244 to the first processor 140, 240 of the rack 1 respectively the medical device 2. In turn, because the low speed data lines 155, 255 are connected to the center tap 144C, 244C of the secondary winding 144B, 244B of the respective transformer 144, 244, no high speed differential signals are transmitted via the lines 155, 255 to the second processor 150, 250 of the rack 1 respectively the medical device 2.

The power sourcing equipment 17 of the rack 1 for feeding power to the medical device 2 is connected via a filter 170 and via lines 171, 172 also to the center taps 144C, 145C of the secondary windings 144B, 145B of the transformers 144, 145, similarly as for the lines 154, 155 of the second communication means 15 of the rack 1 for the low speed communication. On the side of the medical device 2, the power supply 27 via a filter and rectifier 270 and lines 271, 272 is also connected to the center taps 244C, 245C of the secondary windings 244B, 245B of the transformers 244, 245, such that power may be fed from the power sourcing equipment 17 of the rack 1 to the power supply 27 of the medical device 2 using the lines of the high speed differential transmit link TX and the high speed differential receive link RX in common mode, similarly as for establishing the low speed data connection.

Accordingly, as illustrated in Fig. 12, when feeding power over the center taps 144C, 244C of the transformers 144, 244 of the high speed differential transmit link TX a current Ivcc fed over the line 172 is divided and fed over the electrical contacts 111.1A, 211.1A and the electrical contacts 111.1 B, 211.1 B (Ivcc/2 each), hence using the high speed differential transmit link TX in common mode.

Using the high speed differential transmit link TX and the high speed differential receive link RX in common mode for providing the low speed communication and the power feeding bears the additional advantage that the low speed communication and the power feeding may still be functional if one of the lines or electrical contacts 111.1, 211.1 breaks.

For the low speed differential link via the lines 154, 155, 254, 255 only a single pair of lines is used to provide a low speed communication back and forth between the rack 1 and the medical device 2 and, hence, to transmit data back and forth between the rack 1 and the medical device 2.

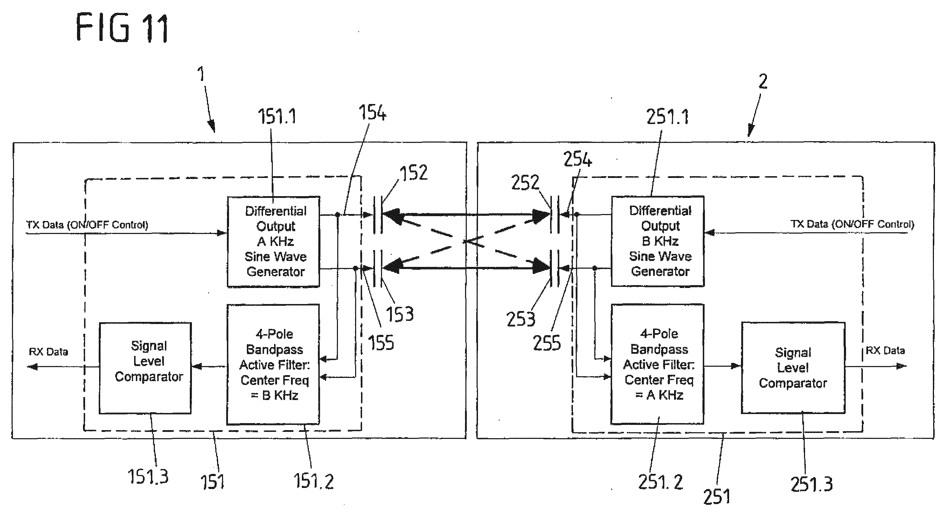

As it is illustrated in Fig. 11, to be able to distinguish between the transmit data transmitted from the rack 1 to the medical device 2 and the receive data transmitted from the medical device 2 to the rack 1, such data are transmitted at different carrier frequencies. For this, the transceiver 151 of the rack 1 in the transmit path comprises a sine wave generator 151.1 to provide a first carrier frequency (in the kHz range) onto which the data to be transmitted form the rack 1 to the medical device 2 is modulated. Similarly, the transceiver 251 of the medical device 2 comprises a sine wave generator 251.1 for producing a sine wave at a second carrier frequency (in the kHz range) which is different from the first carrier frequency and onto which the low speed data to be transmitted from the medical device 2 to the rack 1 is modulated.

Further, each transceiver 151, 251 comprises a bandpass filter 151.2, 251.2, the bandpass filter 151.2 of the transceiver 151 of the rack 1 being centred to the second carrier frequency and the bandpass filter 251.2 of the transceiver 251 of the medical device 2 being centred to the first carrier frequency. And both transceivers 151, 251 comprise a signal level comparator 151.3, 251.3.

For transmitting data from the rack 1 to the medical device 2, the data is modulated onto the first carrier frequency by the sine wave generator 151.1 and is transmitted as a differential data signal via the lines 154, 155, 254, 255 to the transceiver 251 of the medical device 2, in which the differential data signal is filtered out by the bandpass filter 251.2 and output via the signal level comparator 251.3 as receive data to the second processor 250 of the medical device 2.

If in turn data is to be transmitted from the medical device 2 to the rack 1, the data signal is modulated onto the second carrier frequency by the sine wave generator 251.1 of the transceiver 251 of the medical device 2 and is transmitted as a differential signal to the transceiver 151 of the rack 1, in which the received signal is bandpass-filtered by the bandpass filter 151.2 and output via the signal level comparator 151.3 as receive data to the second processor 150 of the rack 1.

Because the transmit data signals and the receive data signals to be transmitted respectively received by the rack 1 are transmitted respectively received at different carrier frequencies with a non-overlapping bandwidth, the signals are readily distinguished, such that they can be transmitted via the same, single pair of lines 154, 155, 254, 255 without any (substantial) interference.

As visible in Fig. 10 and 11, the lines 154, 155, 254, 255 each comprise a capacitor 152, 153, 252, 253. This serves to decouple the second communication means 15, 25 from the direct current connection between the power sourcing equipment 17 and the power supply 27.

In full operation of the medical device 2 in general the major portion of the communication is achieved via the high speed data connection and the high speed communication means 14, 24. In particular, via the high speed communication means 14, 24 in full operation of the medical device 2 control commands and parameters as well as measurement data may be exchanged between the rack 1 and the medical device 2.

The medical device 2 may assume a sleep mode in which most of its functional components are not operational, but are shut down in order to save power. In this sleep mode only the low speed data connection may be operational to provide a low speed communication between the rack 1 and the medical device 2. This is beneficial, because the low speed communication in general consumes much less power than the high speed data communication, such that the low speed communication may run of the battery 26 of the medical device 2.

The low speed communication means 15, 25 may be equipped with an on/off control for transferring the medical device 2 and/or the rack 1 from a sleep mode into a mode of full operation. For this, via signals exchanged via the low speed communication the medical device 2 may be woken up from its sleep mode to switch on all necessary functional components to provide a full operation of the medical device 2.

As indicated in Fig. 10, on the side of the rack 1 as part of the connection element 111 also a detection device 18 in the shape of a Hall sensor may be provided to detect the presence of a medical device 2 at the connection element 111. With the detection device 18 it is checked whether a medical device 2 with its connection element 211 is correctly connected to the connection elements 111 of the rack 1. If yes, an appropriate signal is created to for example initiate communication between the rack 1 and the medical device 2 (high speed communication as well as low speed communication) and in addition to start a power feed from the rack 1 to the medical device 2.

As has been described above with reference to Fig. 7, the connection elements 111, 211 comprise a rotationally symmetrical shape allowing for a connection of the medical device 2 in different engagement positions to the rack 1. For this, the electrical contacts 111.1, 211.1 are evenly spaced about the engagement direction E such that an electrical connection is also achieved when turning the medical device by 180° about the engagement direction E.

Figs. 9A and 9B schematically show how the electrical connection between the medical device 2 and the rack 1 is switched when attaching the medical device 2 in different engagement positions to the rack 1. Fig. 9A herein shows schematically the electrical connection when attaching the medical device 2 in a first engagement position to the rack 1 (denoted direct connection), whereas Fig. 9B illustrates the electrical connection when attaching the medical device 2 in a second engagement position to the rack 1 (denoted reverse connection in which the medical device 2 is turned by 180° with respect to the first engagement position about the engagement direction E).

Fig. 9A and 9B in each case show the (second) connection element 211 of the medical device 2.

In the direct connection, the high speed differential transmit link TX is established via the first pair of contacts 211.1A, 211.1 B, shown at the top in Fig. 9A. Over this first pair of contacts 211.1A, 211.1B also the positive terminal (+) of the power sourcing equipment 17 is connected to the power supply 27 of the medical device 2. Via the second pair of contacts 211.1C, 211.1 D in turn the high speed differential receive link RX is established, and to this second pair of contacts 211.1C, 211.1D the negative terminal (-) of the power sourcing equipment 17 is connected. Between the first pair of contacts 211.1A, 211.1 B and the second pair of contacts 211.1C, 211.1D (each pair in common mode) the low speed differential data link is established.

If the medical device is turned by 180° and connected in the reverse connection to the rack 1, as illustrated in Fig. 9B, the high speed differential transmit link TX now is established via the second pair of contacts 211.1C, 211.1 D, shown at the bottom of Fig. 9B. Via the second pair of contacts 211.1C, 211.1D also the positive terminal (+) of the power sourcing equipment 17 is connected to the power supply 27. The high speed differential receive link RX in this case is established via the first pair of contacts 211.1A, 211.1 B, to which also the negative terminal (-) of the power sourcing equipment 17 is connected. Again, the low speed differential data link is established between the first pair of electrical contacts 211.1A, 211.1 B and the second pair of contacts 211.1C, 211.1 D.

The connection of the medical device 2 in different engagement positions to the rack 1 is also illustrated, with regard to the low speed data connection, in Fig. 11 by the solid lines (direct connection) and the dashed lines (reverse connection) between the capacitors 152, 153, 252, 253.

### List of Reference Numerals

- 1: Rack
- 10: Base part
- 100: Housing
- 100T: Top side
- 101: Power connection
- 102: Power supply unit
- 103: Fixing means (Clamp)
- 104, 105: Connection means
- 106: Communication ports
- 107: Communication means
- 108: Wireless communication interface
- 11: Holding part
- 110: Body (column)
- 110B: Bottom side
- 110P: Protruding section
- 111: First connection element (male connector)
- 111.0: Connection cone
- 111.1: Contact
- 111.2: Groove
- 111.3: Communication port
- 111.4: Front side
- 112: Handle
- 113: Fixing means (Clamp)
- 114, 115: Connection means
- 116: Fastening means
- 117: Battery device
- 118: Wireless communication interface
- 14: First communication means
- 140: First processor (Main CPU)
- 141: Communication interface
- 142A, 142B: Line
- 143A, 143B: Line
- 144, 145: Transformer
- 144A, 144B: Winding
- 144C, 145C: Center tap
- 15: Second communication means
- 150: Second processor (Secondary CPU)
- 151: Transceiver
- 151.1: Sine wave generator
- 151.2: Bandpass filter
- 151.3: Signal level comparator
- 152, 153: Capacitor
- 154, 155: Line
- 16: Battery
- 17: Power sourcing equipment
- 170: Filter
- 171,172: Line
- 18: Detection device (Hall sensor)
- 180: Line
- 2: Medical devices
- 21: Housing
- 210: Indentation
- 211: Second connection element (female connector)
- 211.0: Connection opening
- 211.1, 211.1A, 211.1B, 211.1C, 211.1D: Contact
- 211.2: Engagement device (ball)
- 211.3: Communication port
- 211.4: Pin
- 211.5: Wall
- 211.6: Locking device
- 22: Handle
- 23: Unlocking button
- 24: First communication means
- 240: First processor (Main CPU)
- 241: Communication interface
- 242A, 242B: Line
- 243A, 243B: Line
- 244,245: Transformer
- 244A, 244B: Winding
- 244C, 245C: Center tap
- 25: Second communication means
- 250: Second processor (Secondary CPU)
- 251: Transceiver
- 251.1: Sine wave generator
- 251.2: Bandpass filter
- 251.3: Signal level comparator
- 252, 253: Capacitor
- 254, 255: Line
- 26: Battery
- 27: Power sourcing equipment
- 270: Filter
- 271, 272: Line
- 3: Stand
- 31: Fastening means
- 4: Container
- 42: Line
- A: Rotational symmetry axis
- B: Bed
- E: Engagement direction
- Ivcc: Current
- RX: High speed differential receive link
- TX: High speed differential transmit link

## Claims

1. A rack (1), attachable to a stand, for holding at least one medical device (2), the rack (1) comprising at least
one first connection element (111) to engage with a second connection element (211) of a medical device (2) for connecting the medical device (2) to the rack (1), wherein the at least one first connection element (111) of the rack (1) is connectable with the second connection element (211) of the medical device (2) by attaching the medical device (2) in an engagement direction (E) to the rack (1),
wherein the at least one first connection element (111) of the rack (1) is constituted to establish a mechanical connection for mechanically fixing the medical device (2) to the rack (1) and an electrical connection for electrically connecting the medical device (2) to the rack (1)**characterized in that** the at least one first connection element (111) of the rack (1) is shaped as
- either a connection cone (111.0) protruding from a body (110) of the rack (1) to engage with a connection opening (211.0) of the second connection element (211) of the medical device (2)
- or a connection opening to engage with a connection cone of the second connection element (211) of the medical device (2)
- and that the at least one first connection element (111) of the rack (1) has a rotationally symmetric shape about a rotational symmetry axis (A) directed along the engagement direction (E).

2. The rack (1) according to claim 1, **characterized in that** the at least one first connection element (111) of the rack (1) is constituted to establish the mechanical connection and the electrical connection between the rack (1) and the medical device (2) in at least two different engagement positions of the medical device (2) on the rack (1), the different engagement positions corresponding to different angular positions when turning the medical device (2) about the engagement direction (E).

3. The rack (1) according to one of the preceding claims, **characterized in that** the at least one first connection element (111) of the rack (1) comprises four electrical contacts (111.1).

4. The rack (1) according to claim 3, **characterized in that** the electrical contacts (111.1) are spaced evenly apart about the engagement direction (E).

5. The rack (1) according to one of the preceding claims, **characterized in that** the at least one first connection element (111) of the rack (1) comprises a mechanical locking means (111.2) for mechanically locking the at least one first connection element (111) of the rack (1) to the second connection element (211) of the medical device (2).

6. The rack (1) according to claim 5, **characterized in that** the mechanical locking
means (111.2) is formed by
- a groove (111.2) to engage with at least one engagement element (211.2) of the second connection element (211) of the medical device (2) or
- at least one engagement element to engage with a groove of the second connection element (211) of the medical device (2).

7. The rack (1) according to one of the preceding claims, **characterized in that** the rack (1) comprises a protruding section (110P) extending vertically to the engagement direction (E) on the rack (1) to engage with an associated indentation (210) of the medical device (2).

8. The rack (1) according to claim 7, **characterized in that** the at least one first connection element (111) of the rack (1) is arranged on the protruding section (110P).

9. The rack (1) according to one of the preceding claims, **characterized in that** the at least one first connection element (111) comprising a detection device (111.3) for detecting whether a medical device (2) is connected to the at least one first connection element (111) of the rack (1) or not.

10. A medical device (2) attachable to a rack (1) according to one of claims 1 to 9, the medical device (2) comprising a second connection element (211) to engage with a first connection element (111) of the rack (1) for connecting the medical device (2) to the rack (1), wherein the second connection element (211) of the medical device (2) is connectable with the first connection element (111) of the rack (1) by attaching the medical device (2) in an engagement direction (E) to the rack (1),
wherein
the second connection element (211) of the medical device (1) is constituted to establish a mechanical connection for mechanically fixing the medical device (2) to the rack (1) and an electrical connection for electrically connecting the medical device (2) to the rack (1),
**characterized in that** the second connection element (211) of the medical device (2) is shaped as
- either a connection opening to engage with a connection cone of the first connection element (111) of the rack (1)
- or a connection cone protruding from a body of the medical device (2) to engage with a connection opening (111.0) of the first connection element (111) of the rack (1)
and that the second connection element (211) has a rotationally symmetric shape about a rotational symmetry axis (A) directed along the engagement direction (E).

11. An arrangement of a rack (1) according to one of claims 1 to 9 and a medical device (2) according to claim 10.

12. The arrangement of claim 11, **characterized in that** the first connection element (111) and the second connection element (211) each comprise at least two electrical contacts (111.1, 211.1), wherein in an attached state of the rack (1) and the medical device (2) via the at least two electrical contacts (111.1, 211.1) both a low speed data connection (15, 25) and a high speed data connection (14, 24) between the medical device (2) and the rack (1) is established.

13. The arrangement according to claim 12, **characterized in that** via the at least two electrical contacts (111.1, 211.1) in addition a power supply connection (17, 27), in particular a direct current connection, is established.

## Patentansprüche

1. Gestell (1), das einem Ständer befestigt werden kann, zum Befestigen mindestens eines medizinischen Geräts (2), wobei das Gestell (1) mindestens ein erstes Verbindungselement (111) umfasst, um mit einem zweiten Verbindungselement (211) eines medizinischen Geräts (2) ineinanderzugreifen, um das medizinische Gerät (2) mit dem Gestell (1) zu verbinden, wobei das mindestens eine erste Verbindungselement (111) des Gestells (1) mit dem zweiten Verbindungselement (211) des medizinischen Geräts (2) verbunden werden kann, indem das medizinische Gerät (2) in einer Eingreifrichtung (E) am Gestell (1) angebracht wird,
wobei das mindestens eine erste Verbindungselement (111) des Gestells (1) so konzipiert ist, dass eine mechanische Verbindung zum mechanischen Befestigen des medizinischen Geräts (2) am Gestell (1) und eine elektrische Verbindung zum elektrischen Verbinden des medizinischen Geräts (2) mit dem Gestell (1) hergestellt werden, **dadurch gekennzeichnet, dass** das mindestens eine erste Verbindungselement (111) des Gestells (1) geformt ist als
- entweder ein Verbindungszapfen (111.0), der von einem Körper (110) des Gestells (1) herausragt, um mit einer Verbindungsöffnung (211.0) des zweiten Verbindungselements (211) des medizinischen Geräts (2) ineinanderzugreifen,
- oder eine Verbindungsöffnung, um mit einem Verbindungszapfen des zweiten Verbindungselements (211) des medizinischen Geräts (2) ineinanderzugreifen,
- und dass das mindestens eine erste Verbindungselement (111) des Gestells (1) eine rotationssymmetrische Form um eine Rotationssymmetrieachse (A) aufweist, die entlang der Eingreifrichtung (E) verläuft.

2. Gestell (1) nach Anspruch 1,
**dadurch gekennzeichnet, dass** das mindestens eine erste Verbindungselement (111) des Gestells (1) so konzipiert ist, dass die mechanische Verbindung und die elektrische Verbindung zwischen dem Gestell (1) und dem medizinischen Gerät (2) in mindestens zwei unterschiedlichen Eingreifpositionen des medizinischen Geräts (2) auf dem Gestell (1) hergestellt werden, wobei die unterschiedlichen Eingreifpositionen unterschiedlichen Winkelpositionen beim Drehen des medizinischen Geräts (2) um die Eingreifrichtung (E) entsprechen.

3. Gestell (1) nach einem der vorstehenden Ansprüche,
**dadurch gekennzeichnet, dass** das mindestens eine erste Verbindungselement (111) des Gestells (1) vier elektrische Kontakte umfasst (111.1).

4. Gestell (1) nach Anspruch 3,
**dadurch gekennzeichnet, dass** die elektrischen Kontakte (111.1) gleichmäßig voneinander in Eingreifrichtung (E) beabstandet sind.

5. Gestell (1) nach einem der vorstehenden Ansprüche,
**dadurch gekennzeichnet, dass** das mindestens eine erste Verbindungselement (111) des Gestells (1) ein mechanisches Feststellmittel (111.2) zum mechanischen Feststellen des mindestens einen ersten Verbindungselements (111) des Gestells (1) mit dem zweiten Verbindungselement (211) des medizinischen Geräts (2) umfasst.

6. Gestell (1) nach Anspruch 5,
**dadurch gekennzeichnet, dass** das mechanische Feststellmittel (111.2) gebildet wird durch
- eine Rille (111.2), um mit mindestens einem Eingreifelement (211.2) des zweiten Verbindungselements (211) des medizinischen Geräts (2) ineinanderzugreifen oder
- mindestens ein Eingreifelement, um mit einer Rille des zweiten Verbindungselements (211) des medizinischen Geräts (2) ineinanderzugreifen.

7. Gestell (1) nach einem der vorstehenden Ansprüche,
**dadurch gekennzeichnet, dass** das Gestell (1) einen hervorstehenden Abschnitt (110P) umfasst, der senkrecht zur Eingreifrichtung (E) auf dem Gestell (1) verläuft, um mit einer dazugehörigen Einwölbung (210) des medizinischen Geräts (2) ineinanderzugreifen.

8. Gestell (1) nach Anspruch 7,
**dadurch gekennzeichnet, dass** das mindestens eine erste Verbindungselement (111) des Gestells (1) auf dem hervorstehenden Abschnitt (110P) angeordnet ist.

9. Gestell (1) nach einem der vorstehenden Ansprüche,
**dadurch gekennzeichnet, dass** das mindestens eine erste Verbindungselement (111) eine Erkennungsvorrichtung (111.3) zum Erkennen, ob ein medizinisches Gerät (2) mit dem mindestens einen ersten Verbindungselement (111) des Gestells (1) verbunden ist oder nicht, umfasst.

10. Medizinisches Gerät (2), das an einem Gestell (1) nach einem der Ansprüche 1 bis 9 angebracht werden kann, wobei das medizinische Gerät (2) ein zweites Verbindungselement (211) umfasst, um mit einem ersten Verbindungselement (111) des Gestells (1) ineinanderzugreifen, um das medizinische Gerät (2) mit dem Gestell (1) zu verbinden, wobei das zweite Verbindungselement (211) des medizinischen Geräts (2) mit dem ersten Verbindungselement (111) des Gestells
(1) verbunden werden kann, indem das medizinische Gerät
(2) in einer Eingreifrichtung (E) am Gestell (1) angebracht wird,
wobei
das zweite Verbindungselement (211) des medizinischen Geräts (1) so konzipiert ist, dass eine mechanische Verbindung zum mechanischen Befestigen des medizinischen Geräts (2) am Gestell (1) und eine elektrische Verbindung zum elektrischen Verbinden des medizinischen Geräts (2) mit dem Gestell (1) hergestellt werden,
**dadurch gekennzeichnet, dass** das zweite Verbindungselement (211) des medizinischen Geräts (2) geformt ist als
- entweder eine Verbindungsöffnung, um mit einem Verbindungszapfen des ersten Verbindungselements (111) des Gestells (1) ineinanderzugreifen,
- oder ein Verbindungszapfen, der von einem Körper des medizinischen Geräts (2) herausragt, um mit einer Verbindungsöffnung (111.0) des ersten Verbindungselements (111) des Gestells (1) ineinanderzugreifen,
und dadurch, dass das zweite Verbindungselement (211) eine rotationssymmetrische Form um eine Rotationssymmetrieachse (A) aufweist, die entlang der Eingreifrichtung (E) verläuft.

11. Anordnung eines Gestells (1) nach einem der Ansprüche 1 bis 9 und eines medizinischen Geräts (2) nach Anspruch 10.

12. Anordnung nach Anspruch 11, **dadurch gekennzeichnet, dass** das erste Verbindungselement (111) und das zweite Verbindungselement (211) jeweils mindestens zwei elektrische Kontakte (111.1, 211.1) umfassen, wobei in einem angebrachten Zustand des Gestells (1) und des medizinischen Geräts (2) durch die mindestens zwei elektrischen Kontakte (111.1, 211.1) sowohl eine niedrige Datenaustauschverbindung (15, 25) als auch eine hohe Datenaustauschverbindung (14, 24) zwischen dem medizinischen Gerät (2) und dem Gestell (1) hergestellt werden.

13. Anordnung nach Anspruch 12, **dadurch gekennzeichnet, dass** über die mindestens zwei elektrischen Kontakte (111.1, 211.1) außerdem eine Stromversorgungsverbindung (17, 27), insbesondere eine Gleichstromverbindung, hergestellt wird.

## Revendications

1. Crémaillère (1), pouvant se fixer sur un socle, destinée à maintenir au moins un dispositif (2) médical, la crémaillère (1) comportant au moins un premier élément (111) de liaison pour se mettre en prise avec un second élément (211) de liaison d'un dispositif (2) médical destiné à relier le dispositif (2) médical à la crémaillère (1), dans laquelle ledit au moins un premier élément (111) de liaison de la crémaillère (1) peut être relié au second élément (211) de liaison du dispositif (2) médical par fixation du dispositif (2) médical dans une direction (E) de mise en prise sur la crémaillère (1),
dans laquelle ledit au moins un premier élément (111) de liaison de la crémaillère (1) est constitué de façon à établir une liaison mécanique destinée à fixer mécaniquement le dispositif (2) médical à la crémaillère (1) et une liaison électrique destinée à relier électriquement le dispositif (2) médical à la crémaillère (1) **caractérisée en ce que** ledit au moins un premier élément (111) de liaison de la crémaillère (1) a la forme
- soit d'un cône (111.0) de liaison faisant saillie depuis un corps (110) de la crémaillère (1) pour se mettre en prise avec une ouverture (211.0) de liaison du second élément (211) de liaison du dispositif (2) médical
- soit d'une ouverture de liaison pour se mettre en prise avec un cône de liaison du second élément (211) de liaison du dispositif (2) médical
- et **en ce que** ledit au moins un premier élément (111) de liaison de la crémaillère (1) a une forme symétrique en rotation autour d'un axe (A) de symétrie rotationnel dirigé le long de la direction (E) de mise en prise.

2. Crémaillère (1) selon la revendication 1, **caractérisée en ce que** ledit au moins un premier élément (111) de liaison de la crémaillère (1) est constitué de façon à établir la liaison mécanique et la liaison électrique entre la crémaillère (1) et le dispositif (2) médical dans au moins deux positions de mise en prise différentes du dispositif (2) médical sur la crémaillère (1), les positions de mise en prise différentes correspondant à des positions angulaires différentes lorsque l'on fait tourner le dispositif (2) médical autour de la direction (E) de mise en prise.

3. Crémaillère (1) selon l'une quelconque des revendications précédentes, **caractérisée en ce que** ledit au moins un premier élément (111) de liaison de la crémaillère (1) comporte quatre contacts (111.1) électriques.

4. Crémaillère (1) selon la revendication 3, **caractérisée en ce que** les contacts (111.1) électriques sont espacés uniformément autour de la direction (E) de mise en prise.

5. Crémaillère (1) selon l'une quelconque des revendications précédentes, **caractérisée en ce que** ledit au moins un premier élément (111) de liaison de la crémaillère (1) comporte un moyen (111.2) de verrouillage mécanique destiné à verrouiller mécaniquement ledit au moins un premier élément (111) de liaison de la crémaillère (1) sur le second élément (211) de liaison du dispositif (2) médical.

6. Crémaillère (1) selon la revendication 5, **caractérisée en ce que** le moyen (111.2) de verrouillage mécanique est formé par
- une rainure (111.2) pour se mettre en prise avec au moins un élément (211.2) de mise en prise du second élément (211) de liaison du dispositif (2) médical ou
- au moins un élément de mise en prise pour se mettre en prise avec une rainure du second élément (211) de liaison du dispositif (2) médical.

7. Crémaillère (1) selon l'une quelconque des revendications précédentes, **caractérisée en ce que** la crémaillère (1) comporte une section (110P) faisant saillie qui s'étend verticalement par rapport à la direction (E) de mise en prise sur la crémaillère (1) pour se mettre en prise avec une indentation (210) associée du dispositif (2) médical.

8. Crémaillère (1) selon la revendication 7, **caractérisée en ce que** ledit au moins un premier élément (111) de liaison de la crémaillère (1) est agencé sur la section (110P) faisant saillie.

9. Crémaillère (1) selon l'une quelconque des revendications précédentes, **caractérisée en ce que** ledit au moins un premier élément (111) de liaison comporte un dispositif (111.3) de détection destiné à détecter si un dispositif (2) médical est relié audit au moins un premier élément (111) de liaison de la crémaillère (1) ou non.

10. Dispositif (2) médical pouvant se fixer sur une crémaillère (1) selon l'une quelconque des revendications 1 à 9, le dispositif (2) médical comportant un second élément (211) de liaison pour se mettre en prise avec un premier élément (111) de liaison de la crémaillère (1) destiné à relier le dispositif (2) médical à la crémaillère (1), dans lequel le second élément (211) de liaison du dispositif (2) médical peut être relié au premier élément (111) de liaison de la crémaillère (1) par fixation du dispositif (2) médical dans une direction (E) de mise en prise sur la crémaillère (1),
dans lequel
le second élément (211) de liaison du dispositif (1) médical est constitué de façon à établir une liaison mécanique destinée à fixer mécaniquement le dispositif (2) médical à la crémaillère (1) et une liaison électrique destinée à relier électriquement le dispositif (2) médical à la crémaillère (1),
**caractérisé en ce que** le second élément (211) de liaison du dispositif (2) médical a la forme
- soit d'une ouverture de liaison pour se mettre en prise avec un cône de liaison du premier élément (111) de liaison de la crémaillère (1)
- soit d'un cône de liaison faisant saillie depuis un corps du dispositif (2) médical pour se mettre en prise avec une ouverture (111.0) de liaison du premier élément (111) de liaison de la crémaillère (1)
et **en ce que** le second élément (211) de liaison a une forme symétrique en rotation autour d'un axe (A) de symétrie rotationnel dirigé le long de la direction (E) de mise en prise.

11. Agencement d'une crémaillère (1) selon l'une quelconque des revendications 1 à 9 et d'un dispositif (2) médical selon la revendication 10.

12. Agencement selon la revendication 11, **caractérisé en ce que** le premier élément (111) de liaison et le second élément (211) de liaison comprennent chacun au moins deux contacts (111.1, 211.1) électriques, dans lequel dans un état fixé de la crémaillère (1) et du dispositif (2) médical par l'intermédiaire desdits au moins deux contacts (111.1, 211.1) électriques à la fois une liaison (15, 25) de données à faible vitesse et une liaison (14, 24) de données à vitesse élevée entre le dispositif (2) médical et la crémaillère (1) est établie.

13. Agencement selon la revendication 12, **caractérisé en ce que** par l'intermédiaire desdits au moins deux contacts (111.1, 211.1) électriques additionnels une liaison (17, 27) d'alimentation en courant, en particulier une liaison en courant continu, est établie.
